# FASCICULE DE BREVET EUROPEEN

(11) **EP 0 518 752 B1**
(45) Date de publication et mention de la délivrance du brevet: **17.04.1996**
(21) Numéro de dépôt: 92401590.2
(22) Date de dépôt: 09.06.1992
(51) Int. Cl.: G01N 33/10, G01N 27/22

(54) **Humidimètre pour produits granuleux ou pulvérulents et procédé de mesure du taux d'humidité**
Feuchtigkeitsmessgerät für Granulat oder Pulver und Methode zur Feuchtigkeitsbestimmung
Humidity meter for granular or powdered products and method for measuring moisture content

(30) Priorité: 13.06.1991 FR 9107215
(43) Date de publication de la demande: 16.12.1992
(73) Titulaire: TRIPETTE & RENAUD Société Anonyme:, F-92396 Villeneuve La Garenne Cédex (FR)
(72) Inventeur: Le Gigan, Dominique, F-95620 Parmain (FR)
(74) Mandataire: Chambon, Gérard

(56) Documents cités:
- EP-A- 0 034 459
- US-A- 2 834 201
- MESURES REGULATION AUTOMATISME. vol. 33, no. 1, Janvier 1968, PARIS FR pages 58 - 62; STERU: 'PROCEDE DIELECTRIQUE POUR LA MESURE EN CONTINU DE L'HUMIDITE DES MATERIAUX SOLIDES'

## Description

L'invention concerne un humidimètre pour produits granuleux ou pulvérulents ainsi qu'un procédé de mesure du taux d'humidité.

Il est courant d'utiliser des humidimètres pour les grains de céréales, oléagineux, protéagineux, ou autres produits granuleux ou pulvérulents.

Les appareils connus effectuent généralement des mesures indirectes (par hyperfréquence, infrarouge, diélectrique) et parmi ceux-ci, les humidimètres du type capacitif sont assez satisfaisants.

Les humidimètres qui utilisent la technique de mesure capacitive sont basés sur le fait que le comportement diélectrique du produit à analyser varie en fonction de sa teneur en eau. La cellule de mesure est faite de manière à constituer au moins un condensateur et l'on mesure les différences diélectriques entre l'instant où la cellule est vide et celui où elle est remplie par le produit.

Ces mesures peuvent être réalisées, pour chaque échantillon de produit, à volume constant (celui de la cellule de mesure) ou à poids constant (chaque échantillon ayant un poids déterminé, qui dépend parfois du type de produit).

Pour effectuer ces mesures, le condensateur est par exemple inséré dans un oscillateur, comme il sera précisé plus loin.

Toutefois, les humidimètres, même du type capacitif, ont leurs limites et notamment l'une d'entre elles est la difficulté de corriger l'erreur de mesure liée à l'humidité superficielle du produit à analyser.

En effet, si au cours d'une récolte de grains par exemple, un lot subit une averse avant d'être livré, la mesure effectuée à la réception est entachée d'erreur car l'humidité de surface (eau libre) n'est pas appréciée comme l'humidité interne au grain (eau liée).

Ceci est dû au fait que l'eau liée présente pour la mesure capacitive une constante diélectrique beaucoup plus faible que celle présentée par l'eau libre.

C'est pourquoi on a cherché à corriger les mesures, notamment en faisant des mesures de capacité diélectrique à plusieurs fréquences, ou en adjoignant une mesure de résistivité à la mesure capacitive...

Ces mesures correctives connues présentent certains inconvénients. En particulier, dans le cas de mesures de résistivité ou de conductibilité, celles-ci dépendent notamment de la nature des sels contenus dans l'eau, ce qui peut bien sûr fausser la valeur de ces mesures correctives.

L'invention concerne un humidimètre perfectionné et un nouveau procédé qui permettent d'effectuer ce type de correction, et qui, en particulier, obvient aux inconvénients précités.

L'invention est basée sur la constatation suivante:

L'humidité de l'air est sensible, à une température et une pression données, à la teneur en eau des matériaux qui sont mis en contact avec cet air. En effet, la mise en présence d'un matériau humide dans un gaz sec provoque l'évaporation de l'eau du matériau et l'humidification du gaz.

Dans une enceinte fermée où l'on a fait le vide, cette évaporation se poursuit jusqu'à une limite qui dépend de la température, et pour laquelle la concentration de vapeur d'eau est appelée concentration de saturation. La quantité de vapeur d'eau et sa concentration varient dans le même sens que la température.

Si l'enceinte contient déjà de l'air, les phénomènes sont identiques, mais les délais de saturation sont plus longs.

En conséquence, à une température et une pression barométrique données, un grain humide en présence d'air sec aura tendance à humidifier l'air à son voisinage.

Ce phénomène sera d'autant plus long que l'eau sera fortement liée, et d'autant plus rapide que l'eau sera libre en grande quantité à la surface du grain.

Sur cette base, l'inventeur a pu constater qu'un capteur de mesure hygrométrique plongé dans un lot de grains enregistre une variation d'autant plus importante et rapide que le grain est plus humide en surface.

C'est pourquoi l'humidimètre proposé par l'invention et qui comporte classiquement une cellule pour le produit à analyser et un moyen de mesure indirecte de l'humidité relié à une unité de traitement des mesures, ladite cellule étant adaptée pour être remplie et successivement vidée dudit produit, est remarquable en ce qu'un capteur hygrométrique est aménagé de manière à pouvoir être en contact avec l'air environnant ledit produit à analyser et en ce que ledit capteur est connecté à l'unité de traitement des mesures afin d'apporter des correctifs aux valeurs de l'humidité apparente mesurée par ledit moyen de mesure indirecte.

Selon un mode de réalisation, le capteur hygrométrique est disposé dans la cellule en étant convenablement isolé du produit mais en communication avec l'air ambiant de la cellule.

Toutefois, selon d'autres modes de réalisation, ledit capteur hygrométrique est disposé dans l'une des parois de la cellule ou même à l'extérieur de la cellule mais au voisinage de l'une de ses parois en étant convenablement isolé du produit, mais en communication avec l'air ambiant de la cellule.

De préférence, le capteur hygrométrique est disposé vers la partie inférieure de la cellule.

Selon un autre mode de réalisation comportant une trémie d'alimentation du produit, aménagée au-dessus de la cellule dite principale, le capteur hygrométrique est disposé dans ou sur une cellule annexe qui peut communiquer à volonté avec ladite trémie d'alimentation pour son remplissage avec le produit à analyser, en étant convenablement isolé du produit lorsque celui-ci remplit la cellule annexe mais en communication avec l'air ambiant de ladite cellule annexe, celle-ci comportant des moyens de remplissage distincts de ceux de la cellule principale ainsi que des moyens de vidage.

Pour compléter ou améliorer les mesures et les moyens de correction, un humidimètre selon l'invention peut comporter en outre un capteur de température aménagé dans la cellule de mesure ou la trémie d'alimentation et/ou un capteur de température et/ou un capteur de pression, ces derniers étant aménagés au voisinage du produit à analyser mais en dehors des cellules de mesure. Pour ces deux derniers, il s'agit en effet de capteurs d'ambiance prévus, par exemple, au voisinage de la trémie d'alimentation, et reliés à l'unité de traitement des mesures.

Un humidimètre selon l'invention permet ainsi de réaliser un procédé de mesure du taux d'humidité pour produits granuleux ou pulvérulents qui est remarquable en ce qu'il consiste à effectuer, en plus de la mesure indirecte de l'humidité, une mesure des valeurs hygrométriques, pendant un temps donné, de l'air ambiant de la zone d'analyse, d'une part, en l'absence de produit et, d'autre part, en présence du produit pour créer des valeurs correctives à la valeur de l'humidité apparente mesurée, en évaluant ainsi par les mesures hygrométriques la quantité d'eau libre à la surface du produit.

De préférence, on effectue en même temps des mesures de température et/ou de pression. De cette façon, on peut faire des corrections aussi à partir de ces mesures complémentaires.

Comme il a déjà été dit, les variations de l'hygrométrie liées à l'humidité de surface sont rapides et les diverses mesures effectuées pendant un temps donné permettent donc d'établir une partie des courbes de variation de telle sorte que selon un mode de réalisation de l'invention, les valeurs correctives peuvent être établies par extrapolation de la courbe des valeurs hygrométriques mesurées pendant le temps donné.

Afin de posséder plus de temps pour les mesures hygrométriques, en combinaison avec d'autres moyens mécaniques d'un dispositif complet non spécialement décrit ici, on peut utiliser l'humidimètre précité muni de la cellule annexe et dans ce cas, un procédé de mesure selon l'invention est remarquable en ce qu'il consiste à remplir la cellule annexe préalablement à la cellule principale afin de disposer d'un temps supplémentaire pour les mesures hygrométriques en présence de produit tandis que le vidage de la cellule annexe s'effectue au plus tard avec celui de la cellule principale.

L'invention sera bien comprise et d'autres particularités apparaitront à la lecture de la description qui va suivre et qui se réfère aux dessins annexés dans lesquels:
- les figures 1a à 1c montrent un premier mode de réalisation schématiquement en élévation selon trois phases successives d'une mesure,
- les figures 2a à 2c correspondent aux figures 1a à 1c après rotation de 90°,
- les figures 3a à 3c et 4a à 4c correspondent aux figures respectivement 1a à 1c et 2a à 2c pour un deuxième mode de réalisation,
- les figures 5a à 5d et 6a à 6d correspondent de la même manière (avec deux figures supplémentaires) aux figures précédentes pour un troisième mode de réalisation.

Sur toutes les figures, on peut voir une trémie d'alimentation 1 pour un produit 2 à analyser, tel que des grains de céréales.

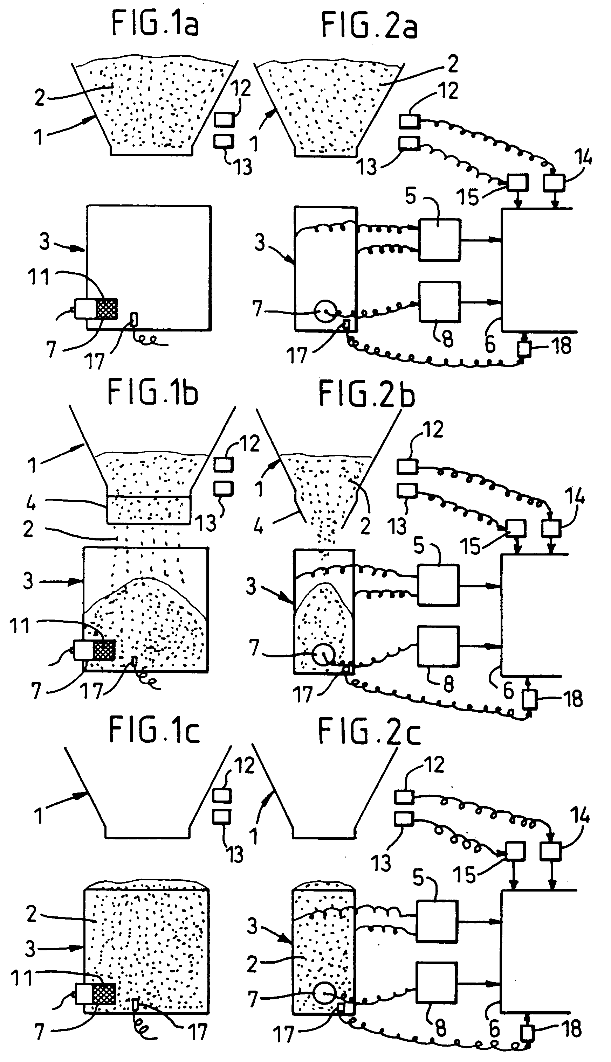

Cette trémie d'alimentation 1 est disposée juste au-dessus d'une cellule de mesure 3 qu'elle peut remplir par l'ouverture d'un volet 4 dont elle est munie (figures 1b, 2b, 3b, 4b, 5c, 6c).

Un moyen de mesure indirecte du taux d'humidité est prévu, ici par effet capacitif, deux parois opposées de la cellule 3 formant les armatures d'un condensateur, ces deux parois étant en outre connectées par l'intermédiaire d'une interface convenable 5, à une unité 6 électronique de traitement des mesures (figures 2a à 2c, 4a à 4c, 6a à 6d).

Tous les modes de réalisation sont en outre pourvus d'un capteur hygrométrique 7, 7**′** (7 dans le premier mode de réalisation représenté et 7′ dans les autres), qui est également relié à l'unité de traitement 6 par une interface 8 (figures 2a à 2c, 4a à 4c, 6a à 6d).

L'unité de traitement 6 est conçue en fonction du type de la mesure utilisée, avantageusement ici du type capacitif, dont le principe a été rappelé au début. L'unité peut ainsi comporter un oscillateur dont on peut mesurer la dérive en fréquence ou bien l'atténuation du signal à fréquence fixe, lorsque le produit est introduit, un microprocesseur étant prévu pour traiter ces informations. Pour chaque type de produit, un calibrage de l'appareil permet de relier le résultat des mesures (variations de la fréquence ou de l'amplitude) à la teneur en eau dudit produit. L'interface 5 est un module électronique destiné à mettre en forme et éventuellement convertir le signal reçu tandis que l'interface 8 dépend bien sûr du type de capteur hygrométrique choisi, comme il sera précisé ci-après.

Dans le mode de réalisation des figures 1a, 1b, 1c et 2a, 2b, 2c, le capteur hygrométrique 7 est disposé dans la partie inférieure de la cellule 3.

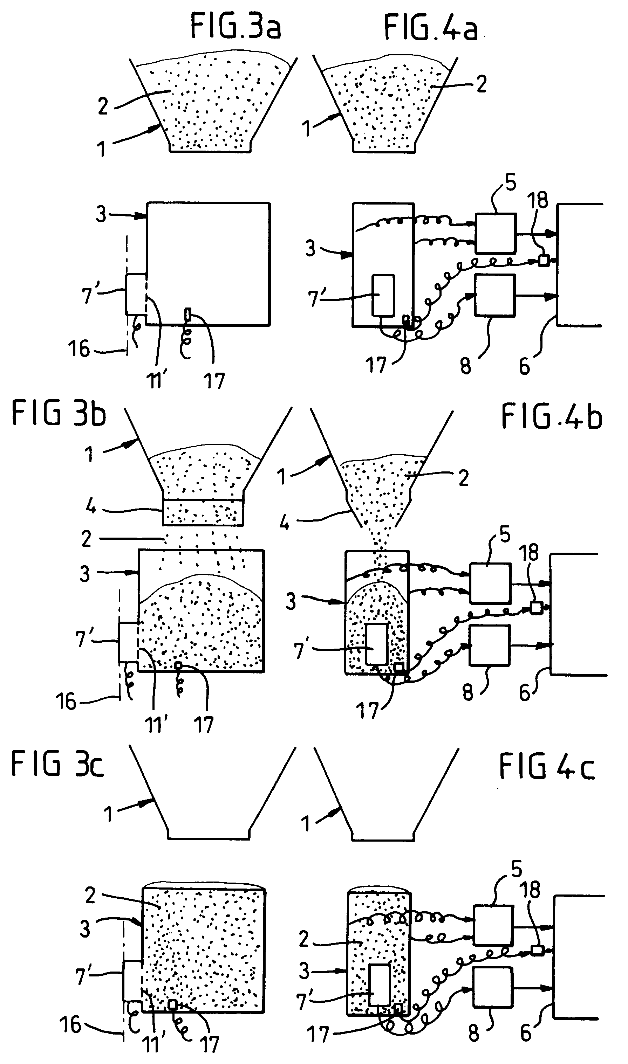

Dans le mode de réalisation des figures 3a, 3b, 3c, et 4a, 4b, 4c, le capteur 7**′** est aménagé à l'extérieur de la cellule 3 sur l'une des parois de celle-ci, mais aussi vers la partie inférieure de ladite cellule 3. Dans ce mode de réalisation, on doit toutefois admettre que le capteur 7′ peut être aménagé dans l'épaisseur de l'une des parois de la cellule, si cette épaisseur le permet (paroi épaisse schématisée en trait interrompu 16 sur les figures 3a à 3c), ou au contraire être à une distance plus importante que celle représentée étant entendu cependant qu'il reste en contact avec l'air environnant le produit à analyser comme il sera précisé ci-après.

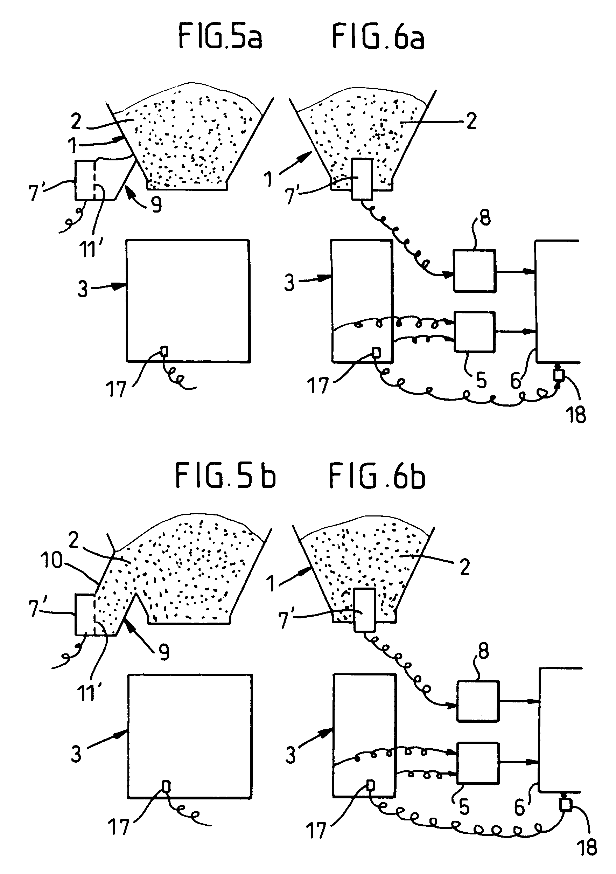

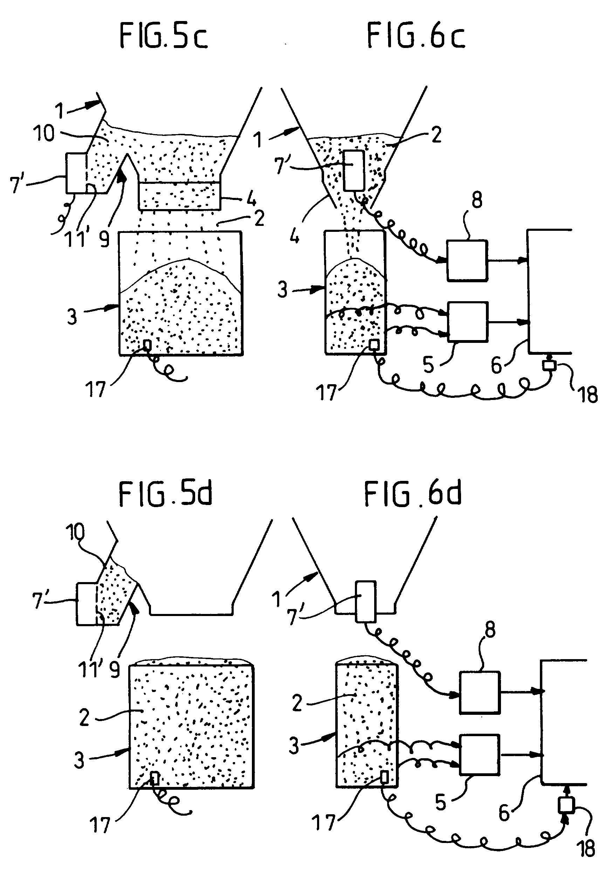

Dans le mode de réalisation des figures 5a à 5d et 6a à 6d, le capteur 7′ ( de conception semblable à celui du mode de réalisation précédent et qui porte la même référence) est aménagé sur une cellule annexe 9 (figures 5a à 5d), laquelle peut communiquer avec la trémie d'alimentation 1 pour son remplissage, par exemple par un volet 10 (figures 5b à 5d). Le capteur 7′ pourrait bien sûr être du type de celui référencé 7 du premier mode de réalisation et disposé dans la cellule annexe 9.

Comme le montrent bien les dessins, le capteur 7, 7′ est dans tous les cas isolé du produit à analyser par un filtre 11 ou similaire (figures 1a à 1c et 2a à 2c) ou 11′ (toutes les autres figures).

En effet, il est clair, comme déjà dit, que le capteur 7, 7′ est bien destiné à établir une mesure d'hygrométrie de l'air en présence du produit (et sans produit comme il sera expliqué ci-après) et on cherche donc à éviter tout contact des parties sensibles du capteur avec le produit lui-même.

Il existe de nombreux types de capteurs hygrométriques. Certains hygromètres sont pourvus de cheveux ou de fibres synthétiques de polymères et fonctionnent par allongement. On connaît aussi les hygromètres du type capacitif munis d'un condensateur en forme de film et dont la valeur du diélectrique varie en fonction du taux de la vapeur d'eau présente. On peut également baser la mesure sur l'impédance qui varie selon la teneur en eau. Il existe encore les capteurs hygrométriques dits à "point de rosée" pourvus d'un miroir refroidi ou d'une sonde d'alumine.

Pour des problèmes de temps de réponses, de dimension, de coût et d'interfaçage, les capteurs hygrométriques du type capacitif ou à impédance conviennent parfaitement.

Par ailleurs, il est possible d'adjoindre des capteurs pour des mesures complémentaires, tels que des capteurs d'ambiance, comme les capteurs 12 et 13 respectivement de température et de pression représentés à titre d'exemple sur les figures 1a à 1c et 2a à 2c. Les capteurs 12 et 13 sont ici naturellement reliés à l'unité de traitement 6 par des interfaces respectivement 14 et 15. Ces capteurs qui analysent les conditions de la mesure peuvent être disposés à de nombreux endroits en dehors de la cellule 3, le capteur 13 de pression n'étant d'ailleurs pas véritablement nécessaire pour ce type de mesure. La température du grain joue sur la valeur diélectrique et un capteur de température est généralement prévu dans la cellule 3 (voire dans la trémie 1) tel que le capteur schématisé en 17 sur les figures, ledit capteur étant relié à l'unité de traitement 6 par une interface 18.

Un humidimètre de ce type comporte évidemment de nombreux autres moyens non représentés, afin d'assurer les différentes phases opératoires, notamment de remplissage et de vidage des cellules et de la trémie, et à cet effet, on comprend bien que la cellule 3 et la cellule annexe 9 sont évidemment pourvues de moyens convenables (non représentés) pour leur vidage tels que des volets, par exemple du type du volet 4 de la trémie 1.

Un tel humidimètre permet d'effectuer un procédé tel que mentionné ci-avant et dont les phases diverses sont représentées sur les dessins.

Avec les modes de réalisation des figures 1a à 1e, 2a à 2c d'une part, et 3a à 3c, 4a à 4c d'autre part, on procède de la manière suivante.

On effectue d'abord une mesure hygrométrique en l'absence de produit au moyen du capteur 7, 7′ (figures 1a, 2a, 3a, 4a), pendant un temps donné. On effectue ensuite dans la cellule 3 une mesure de l'hygrométrie en présence de produit (figures 1b, 2b, 3b, 4b ou 1c, 2c, 3c, 4c) ainsi qu'une mesure indirecte de l'humidité (par exemple par effet capacitif) lorsque la cellule 3 est remplie (figures 1c, 2c, 3c, 4c).

On comprend que la position du capteur 7, 7′ en bas de la cellule 3 permet d'accroître la durée dont on dispose pour effectuer la mesure hygrométrique en présence du produit sans augmenter pour autant la durée totale du pocédé.

Ces mesures permettent d'effectuer les corrections dont il a déjà été question, tandis que les capteurs 12 et 13 établissent pendant ce temps des mesures d'ambiance.

Si on se réfère maintenant aux figures 5a à 5d et 6a à 6d, le procédé est sensiblement le même, mis à part le fait que pour effectuer la mesure d'hygrométrie en présence de produit, on remplit d'abord la cellule annexe 9 (figure 5b, 6b), les figures 5a, 6a correspondant aux mesures à vide, les figures 5c à 6c au remplissage (avec éventuellement toujours les mesures d'hygrométrie en cours) et les figures 5d et 6d aux mesures effectives d'humidité (après remplissage). On constate toutefois qu'avec un mode de réalisation selon les figures 5a à 5d et 6a à 6d, on peut disposer d'un temps de mesure hygrométrique en présence du produit particulièrement appréciable puisque la cellule 9 peut être remplie (par le volet 10) très tôt.

Ensuite, bien sûr, la cellule 3 est vidée, comme déjà dit, de même que la cellule annexe 9 (cette dernière pouvant l'être par exemple simultanément avec la cellule 3 ou juste avant).

De très nombreux moyens peuvent en outre être utilisés avec ce type d'appareil, moyens classiques ou faisant l'objet d'autres brevets en cours, tels que des moyens d'arasage de la cellule, des moyens de remplissage et de vidage, etc.

## Revendications

1. Humidimètre pour produits (2) granuleux ou pulvérulents comportant une cellule (3) pour le produit à analyser et un moyen de mesure indirecte de l'humidité relié à une unité de traitement (6) des mesures, ladite cellule (3) étant adaptée pour être remplie et successivement vidée dudit produit, caractérisé en ce qu'un capteur hygrométrique (7,7′) est aménagé de manière à pouvoir être en contact avec l'air environnant ledit produit à analyser et en ce que ledit capteur (7,7′) est connecté à l'unité (6) de traitement des mesures afin d'apporter des correctifs aux valeurs de l'humidité apparente mesurée par ledit moyen de mesure indirecte.

2. Humidimètre selon la revendication 1, caractérisé en ce que le capteur hygrométrique (7) est disposé dans la cellule (3) en étant convenablement isolé du produit mais en communication avec l'air ambiant de la cellule.

3. Humidimètre selon la revendication 1, caractérisé en ce que le capteur hygrométrique (7′) est disposé dans l'une des parois de la cellule (3) en étant convenablement isolé du produit, mais en communication avec l'air ambiant de la cellule.

4. Humidimètre selon la revendication 1, caractérisé en ce que le capteur hygrométrique (7′) est disposé à l'extérieur de la cellule (3) mais au voisinage de l'une de ses parois en étant convenablement isolé du produit, mais en communication avec l'air ambiant de la cellule.

5. Humidimètre selon l'une des revendications 1 à 4, caractérisé en ce que le capteur hygrométrique (7,7′) est disposé vers la partie inférieure de la cellule.

6. Humidimètre selon la revendication 1, et comportant une trémie d'alimentation (1) du produit aménagée au-dessus de la cellule (3) dite principale, caractérisé en ce que le capteur hygrométrique (7′) est disposé dans ou sur une cellule annexe (9) qui peut communiquer à volonté avec ladite trémie d'alimentation (1) pour son remplissage avec le produit à analyser, en étant convenablement isolé du produit lorsque celui-ci remplit la cellule annexe (9) mais en communication avec l'air ambiant de ladite cellule annexe, celle-ci comportant des moyens de remplissage (10) distincts de ceux de la cellule principale (3) ainsi que des moyens de vidage.

7. Humidimètre selon l'une des revendications 1 à 6, caractérisé en ce qu'il comporte en outre un capteur de température (7) aménagé dans la cellule (3) ou la trémie d'alimentation (1) et/ou un capteur de température (12) et/ou un capteur de pression (13), ces deux derniers étant aménagés au voisinage du produit à analyser mais en dehors des cellules de mesure (3,9).

8. Procédé de mesure du taux d'humidité pour produits granuleux ou pulvérulents au moyen d'un humidimètre conforme aux revendications 1 à 7, caractérisé en ce qu'il consiste à effectuer, en plus de la mesure indirecte de l'humidité, une mesure des valeurs hygrométriques, pendant un temps donné, de l'air ambiant de la zone d'analyse, d'une part, en l'absence de produit et, d'autre part, en présence du produit pour créer des valeurs correctives à la valeur de l'humidité apparente mesurée, en évaluant ainsi par les mesures hygrométriques la quantité d'eau libre à la surface du produit.

9. Procédé selon la revendication 8, caractérisé en ce qu'il consiste à établir en même temps des mesures de température et/ou de pression.

10. Procédé selon l'une des revendications 8 et 9, caractérisé en ce que les valeurs correctives sont établies par extrapolation de la courbe des valeurs hygrométriques mesurées pendant le temps donné.

11. Procédé selon l'une des revendications 8 à 10, au moyen d'un humidimètre selon la revendication 6, caractérisé en ce qu'il consiste à remplir la cellule annexe préalablement à la cellule principale afin de disposer d'un temps supplémentaire pour les mesures hygrométriques en présence de produit tandis que le vidage de la cellule annexe s'effectue au plus tard avec celui de la cellule principale.

## Claims

1. Moisture measuring device for granular or powdered products (2) comprising a cell (3) for the product to be analysed and a means for indirect measurement of the moisture connected to a unit (6) for processing the measurements, said cell (3) being adapted to be filled and successively emptied of said product, characterised in that a hygrometric sensor (7, 7') is arranged so as to be able to be in contact with the air surrounding said product to be analysed and in that said sensor (7, 7') is connected to the unit (6) for processing the measurements in order to make corrections to the apparent moisture values measured by said means for indirect measurement.

2. Moisture measuring device according to claim 1, characterised in that the hygrometric sensor (7) is disposed in the cell (3) while being suitably isolated from the product but in communication with the ambient air of the cell.

3. Moisture measuring device according to claim 1, characterised in that the hygrometric sensor (7') is disposed in one of the walls of the cell (3) while being suitably isolated from the product but in communication with the ambient air of the cell.

4. Moisture measuring device according to claim 1, characterised in that the hygrometric sensor (7') is disposed outside the cell (3) but in the vicinity of one of its walls while being suitably isolated from the product but in communication with the ambient air of the cell.

5. Moisture measuring device according to one of claims 1 to 4, characterised in that the hygrometric sensor (7, 7') is disposed towards the lower part of the cell.

6. Moisture measuring device according to claim 1 and comprising a feed hopper (1) for the product arranged above the so-called principal cell (3), characterised in that the hygrometric sensor (7') is disposed in or on a subsidiary cell (9) which can communicate at will with said feed hopper (1) for filling with the product to be analysed while being suitably isolated from the product when the latter fills the subsidiary cell (9) but in communication with the ambient air of said subsidiary cell, the latter comprising filling means (10) separate from those of the principal cell (3) and emptying means.

7. Moisture measuring device according to one of claims 1 to 6, characterised in that it additionally comprises a temperature sensor (7) arranged in the cell (3) or the feed hopper (1) and/or a temperature sensor (12) and/or a pressure sensor (13), the last two being arranged in the vicinity of the product to be analysed but outside the measuring cells (3, 9).

8. Method for measuring the moisture content for granular or powdered products by means of a moisture measuring device in accordance with claims 1 to 7, characterised in that in addition to the indirect measurement of the moisture, it consists in measuring the hygrometric values of the ambient air of the analysis zone on the one hand in the absence of product and on the other in the presence of product for a given time in order to create correction values for the measured apparent moisture value, thus evaluating the quantity of free water on the surface of the product through the hygrometric measurements.

9. Method according to claim 8, characterised in that it consists in taking temperature and/or pressure measurements at the same time.

10. Method according to one of claims 8 and 9, characterised in that the corrective values are established by extrapolation of the curve of the hygrometric values measured during the given time.

11. Method according to one of claims 8 to 10, by means of a moisture measuring device according to claim 6, characterised in that it consists in filling the subsidiary cell before the principal cell in order to have an additional time for the hygrometric measurements in the presence of product while the emptying of the subsidiary cell is effected later with that of the principal cell.

## Patentansprüche

1. Feuchtigkeitsmeßgerät für granulat- oder pulverförmige Erzeugnisse (2) mit einer Zelle (3) für das zu analysierende Erzeugnis und einem Mittel zur indirekten Messung der Feuchtigkeit, das mit einer Einheit (6) zur Verarbeitung der Messungen verbunden ist, wobei die besagte Zelle (3) so ausgebildet ist, daß sie mit dem besagten Erzeugnis gefüllt und nach und nach von ihm entleert werden kann, dadurch gekennzeichnet, daß ein hygrometrischer Meßfühler (7, 7') derart angeordnet ist, daß er mit der Umgebungsluft des besagten, zu analysierenden Erzeugnisses in Kontakt geraten kann und daß der besagte Meßfühler (7, 7') mit der Einheit (6) zur Verarbeitung der Messungen verbunden ist, um an den von dem besagten Mittel zur indirekten Messung gemessenen Werten der scheinbaren Feuchtigkeit Korrekturen vorzunehmen.

2. Feuchtigkeitsmeßgerät nach Anspruch 1, dadurch gekennzeichnet, daß der hygrometrische Meßfühler (7) derart in der Zelle (3) angeordnet ist, daß er in geeigneter Weise vom Erzeugnis getrennt ist, aber mit der Umgebungsluft der Zelle in Verbindung steht.

3. Feuchtigkeitsmeßgerät nach Anspruch 1, dadurch gekennzeichnet, daß der hygrometrische Meßfühler (7') derart in einer der Wände der Zelle (3) angeordnet ist, daß er in geeigneter Weise vom Erzeugnis getrennt ist, aber mit der Umgebungsluft der Zelle in Verbindung steht.

4. Feuchtigkeitsmeßgerät nach Anspruch 1, dadurch gekennzeichnet, daß der hygrometrische Meßfühler (7') außerhalb der Zelle (3), aber in der Nähe einer ihrer Wände derart angeordnet ist, daß er in geeigneter Weise vom Erzeugnis getrennt ist, aber mit der Umgebungsluft der Zelle in Verbindung steht.

5. Feuchtigkeitsmeßgerät nach einem der Ansprüche 1 bis 4, dadurch gekennzeichnet, daß der hygrometrische Meßfühler (7, 7') in Richtung zum Unterteil der Zelle hin angeordnet ist.

6. Feuchtigkeitsmeßgerät nach Anspruch 1 mit einem Fülltrichter (1) für das Erzeugnis, der oberhalb der, Hauptzelle genannten, Zelle (3) angeordnet ist, dadurch gekennzeichnet, daß der hygrometrische Meßfühler (7') in oder an einer Nebenzelle (9), die nach Belieben mit dem besagten Fülltrichter (1) zu ihrer Füllung mit dem zu analysierenden Erzeugnis in Verbindung treten kann, derart angeordnet ist, daß er in geeigneter Weise vom Erzeugnis getrennt ist, wenn dieses in die Nebenzelle (9) eingefüllt ist, aber mit der Umgebungsluft der besagten Nebenzelle in Verbindung steht, wobei letztere Füllmittel (10), die von denjenigen der Hauptzelle (3) verschieden sind, sowie Entleerungsmittel aufweist.

7. Feuchtigkeitsmeßgerät nach einem der Ansprüche 1 bis 6, dadurch gekennzeichnet, daß es außerdem einen Temperaturmeßfühler (7), der in der Zelle (3) oder im Fülltrichter (1) angeordnet ist, und/oder einen Temperaturmeßfühler (12) und/oder einen Druckmeßfühler (13) aufweist, wobei diese beiden letzteren in der Nähe des zu analysierenden Erzeugnisses aber außerhalb der Meßzellen (3, 9) angeordnet sind.

8. Verfahren zur Messung des Feuchtigkeitsgehalts von granulat- oder pulverförmigen Erzeugnissen mittels eines Feuchtigkeitsmeßgerätes nach den Ansprüchen 1 bis 7, dadurch gekennzeichnet, daß es darin besteht, zusätzlich zur indirekten Messung der Feuchtigkeit eine Messung der hygrometrischen Werte der Umgebungsluft der Analysenzone während eines vorgegebenen Zeitraums durchzuführen, und zwar einerseits in Abwesenheit des Erzeugnisses und andererseits in Gegenwart des Erzeugnisses zur Bildung von Korrekturwerten für den gemessenen Wert der scheinbaren Feuchtigkeit, indem auf diese Weise durch die hygrometrischen Messungen die Menge an freiem Wasser auf der Oberfläche des Erzeugnisses ermittelt wird.

9. Verfahren nach Anspruch 8, dadurch gekennzeichnet, daß es darin besteht, zur gleichen Zeit Messungen der Temperatur und/oder des Druckes durchzuführen.

10. Verfahren nach einem der Ansprüche 8 oder 9, dadurch gekennzeichnet, daß die Korrekturwerte durch Extrapolation der Kurve der während des vorgegebenen Zeitraums gemessenen hygrometrischen Werte gebildet werden.

11. Verfahren nach einem der Ansprüche 8 bis 10 mittels eines Feuchtigkeitsmeßgerätes nach Anspruch 6, dadurch gekennzeichnet, daß es darin besteht, die Nebenzelle vor der Hauptzelle zu füllen, um über einen zusätzlichen Zeitraum für die hygrometrischen Messungen in Gegenwart des Erzeugnisses zu verfügen, während die Entleerung der Nebenzelle spätestens mit derjenigen der Hauptzelle durchgeführt wird.
